# EUROPEAN PATENT SPECIFICATION

(11) **EP 1 591 538 B1**
(45) Date of publication and mention of the grant of the patent: **01.09.2010**
(21) Application number: 05007987.0
(22) Date of filing: 12.04.2005
(51) Int. Cl.: C12Q 1/68

(54) **Polynucleotide probe having enhanced binding specificity, microarray having the probe immobilized thereon, and method of designing the probe**
Polynukleotidsonden mit verbesserter Spezifität, Microarrays welche solche Sonden beinhalten, sowie Methode für den Entwurf solcher Sonden
Sonde polynucléotidique presentant une specificité accrue, puce à ADN la comprenant, et procédé de conception de ces sondes.

(30) Priority: 30.04.2004 KR 2004030447
(43) Date of publication of application: 02.11.2005
(73) Proprietor: SAMSUNG ELECTRONICS CO., LTD., Suwon-si, Gyeonggi-do (KR)
(72) Inventor: Oh, Ji-young, Yeongtong-gu Suwon-si, Gyeonggi-do (KR); Huh, Nam, Seoul (KR)
(74) Representative: Grünecker, Kinkeldey, Stockmair & Schwanhäusser Anwaltssozietät

(56) References cited:
- PIECHOCKI MARIE P ET AL: "Oligonucleotide design and optimized protocol for site-directed mutagenesis" BIOTECHNIQUES, vol. 16, no. 4, 1994, pages 702-707, XP009053162 ISSN: 0736-6205
- STRATAGENE: "QuikChange Site-Directed Mutagenesis Kit"[Online] XP002342578 www.stratagene.com Retrieved from the Internet: URL:http://web.archive.org/web/20030413070 908/http://www.stratagene.com/manuals/2005 18.pdf> [retrieved on 2003-04-13]
- K[MPKE T KIENINGER M MECKLENBURG M: "Efficient primer design algorithms" BIOINFORMATICS, OXFORD UNIVERSITY PRESS, OXFORD,, GB, vol. 17, no. 3, March 2001 (2001-03), pages 214-225, XP002959105 ISSN: 1367-4803
- MITSUHASHI M ET AL: "OLIGONUCLEOTIDE PROBE DESIGN - A NEW APPROACH" NATURE, MACMILLAN JOURNALS LTD. LONDON, GB, vol. 367, 24 February 1994 (1994-02-24), pages 759-761, XP002948495 ISSN: 0028-0836

## Description

### BACKGROUND OF THE INVENTION

### 1. Field of the Invention

The present invention relates to a method of designing an oligonucleotide probe having enhanced binding specificity to a target nucleic acid.

### 2. Description of the Related Art

A "polynucleotide microarray" is a microarray in which polynucleotide groups are densely immobilized in discrete known regions on a substrate. Such a microarray is well known in the art. Regarding the microarray, see, for example, U.S. Patent Nos. 5,445,934 and 5,744,305. Generally, a polynucleotide immobilized on a substrate specifically binds to a nucleic acid to be analyzed, for example, hybridizes with the nucleic acid and the target nucleic acid can be analyzed through the specific binding. Such a polynucleotide immobilized on a substrate and hybridizing with a target nucleic acid is called a probe polynucleotide (or oligonucleotide).

Conventionally, a polynucleotide probe was generally designed considering thermodynamic variables including a temperature to hybridize with a target nucleic acid, a length, the formation of a homodimer in a molecule, the presence of a sequence homology with another polynucleotide probe, and a position of a target site. Among these, the thermodynamic variable is to consider the hybridization energy between a probe sequence and the target nucleic acid and a probe having a hybridization temperature in a certain range is selected. When the hybridization temperature is high, specific binding increases, and when the hybridization temperature is low, nonspecific binding increases. Thus, in a conventional process of designing a probe, the hybridization temperature was arbitrarily selected in a proper range. The length of a probe is generally 15-50 bp, but is not limited thereto. It is not preferable that a homodimer is formed in a probe molecule or there is sequence homology with other probe and it is preferable to have a sequence which avoids these cases. Further, it is preferable that the target site is generally located so as to correspond to the center of a probe in view of steric effect, and the like.

However, according to a conventional method of selecting a polynucleotide probe, the hybridization temperature was considered in the entire probe. Thus, although the hybridization temperatures of the total probe were equal or similar, there is a possibility of nonspecific binding when hybridization temperatures of an oligonucleotide at 5' end side based on a nucleotide corresponding to a target site with target sequence were considerably different from those of an oligonucleotide at 3' end side based on a nucleotide corresponding to a target site with target sequence.

While intensively studying to resolve the above problem, the inventors discovered that when a hybridization temperature of a target nucleic acid and a polynucleotide sequence including the first nucleotide in the direction of 5' end from a nucleotide corresponding to a target site through 5' end nucleotide and a hybridization temperature of the target nucleic acid and a polynucleotide sequence including the first nucleotide in the direction of 3' end from the nucleotide corresponding to the target site through 3' end nucleotide are similar or identical to each other, the nonspecific binding can be considerably reduced, thereby completing the present invention.

### SUMMARY OF THE INVENTION

The present invention provides a method of designing the polynucleotide probe having enhanced binding specificity.

According to the present invention, there is provided a method of designing an oligonucleotide probe having enhanced binding specificity to a target site according to claim 1, the method including: calculating a difference between a hybridization temperature (Tm) of an oligonucleotide sequence including the first nucleotide in the direction of 5' end from a nucleotide corresponding to the target site through 5' end nucleotide and an oligonucleotide sequence complementary thereto and a hybridization temperature (Tm) of an oligonucleotide sequence including the first nucleotide in the direction of 3' end from the nucleotide corresponding to the target site through 3' end nucleotide and an oligonucleotide sequence complementary thereto; and selecting an oligonucleotide probe having the difference of not greater than 5°C.

Further provided is a method of preparing an oligonucleotide probe according to claim 2.

### BRIEF DESCRIPTION OF THE DRAWINGS

The above and other features and advantages of the present invention will become more apparent by describing in detail exemplary embodiments thereof with reference to the attached drawings in which:
FIG. 1A illustrates a wild type and a mutant type oligonucleotide probes designed according to a conventional method when a nucleotide site complementary to the 342^{nd} C of a target sample E01-11 (SEQ ID NO. 9) is used as a target site;
FIG. 1B illustrates a wild type and a mutant type oligonucleotide probes designed according to a method of the present invention when the nucleotide site complementary to the 342^{nd} C of the target sample E01-11 (SEQ ID NO. 9) is used as a target site;
FIG. 2A illustrates a wild type and a mutant type oligonucleotide probes designed according to the conventional method when a nucleotide site complementary to the 147th C of a target sample E02-22 (SEQ ID NO. 10) is used as a target site;
FIG. 2B illustrates a wild type and a mutant type oligonucleotide probes designed according to the method of the present invention when the nucleotide site complementary to the 147th C of the target sample E02-22 (SEQ ID NO. 10) is used as a target site;
FIG. 3A is a MA graph illustrating the results of hybridizing genome DNA derived from a normal human on a control microarray on which probes WP1 and MP1 designed according to the conventional method are immobilized;
FIG. 3B is a MA graph illustrating the results of hybridizing a target nucleic acid on a microarray on which probes WP2 and MP2 designed according to the method of the present invention are immobilized;
FIG. 4A is a MA graph illustrating the results of hybridizing genome DNA derived from a normal human on a control microarray on which probes WP3 and MP3 designed according to the conventional method are immobilized; and
FIG. 4B a MA graph illustrating the results of hybridizing a target nucleic acid on a microarray on which probes WP4 and MP4 designed according to the method of the present invention are immobilized.

### DETAILED DESCRIPTION OF THE INVENTION

In the present invention, the oligonucleotide probe is a hybridization probe used to analyze the type of gene of a target nucleic acid using a degree of a hybridization reaction that is obtained after performing the hybridization reaction with the target nucleic acid. In the present invention, the target nucleic acid is a nucleic acid or a nucleic acid sample having a target site to be analyzed. The target site is a position of a certain nucleotide of which a variation is found in a target nucleic acid. Examples of the variation include allelic variation, single nucleotide polymorphism (SNP), etc., but are not limited thereto. Also, the cause of the variation includes natural and artificial mutations, and the variation used in the present invention is not particularly limited to its cause.

In the present invention, the hybridization temperature (Tm) is a temperature at which a double stranded polynucleotide corresponding to a half of maximum value of a double stranded polynucleotide, which is obtained when two strands of a polynucleotide are hybridized by annealing, is obtained or a half of a double stranded polynucleotide is dissociated when the double stranded polynucleotide is dissociated by heat. The hybridization temperature is dependent on an amount of base in a polynucleotide, a length, a concentration of a salt in a solution, and the like. The hybridization temperature (Tm) herein is a temperature under a solution condition commonly used in the art.

In the oligonucleotide probe designed or prepared according to the method of the present invention, the hybridization temperature of an oligonucleotide sequence in the direction of 5' end, excluding a nucleotide corresponding to a target site, and an oligonucleotide sequence of a target nucleic acid corresponding thereto and the hybridization temperature of an oligonucleotide sequence in the direction of 3' end, excluding a nucleotide corresponding to a target site, and a oligonucleotide sequence of a target nucleic acid corresponding thereto are similar to each other. A difference in the hybridization temperature is preferably 0-7°C, and more preferably 0-5°C. As long as the difference in the hybridization temperature is within the above range, the length of the oligonucleotide in the direction of 5' end and the length of the oligonucleotide in the direction of 3' end may be identical or different. However, an oligonucleotide designed such that the target site corresponds to the vicinity of the central of the oligonucleotide is generally used. Thus, the length of the oligonucleotide in the direction of 5' end and the length of the oligonucleotide in the direction of 3' end are similar or identical to each other.

In the present invention, the length of the oligonucleotide is in a range commonly used in the art and is not particularly limited. The length of the oligonucleotide is, for example, 15-50 bp, and preferably 15-40 bp, and more preferably 20-30 bp.

The oligonucleotide probe prepared according to the method of the present invention can be effectively used to analyze a target nucleic acid by being immobilized on a DNA microarray. Oligonucleotide probes of the present invention immobilized on a DNA microarray have similar hybridization temperatures to each other, thereby improving the results of hybridization reactions obtained using the microarray.

In the probe immobilized on the microarray, a difference between a hybridization temperature of a wild type probe (wp) perfectly matching to a wild type target nucleic acid and a sequence complementary thereto and a hybridization temperature of a mutant type probe (mp) perfectly matching to a mutant type target nucleic acid and a sequence complementary thereto may also be in a range of 0-5°C.

Another embodiment of the present invention is to provide a method of designing an oligonucleotide probe having enhanced binding specificity to a target site according to claim 1, the method including: calculating a difference between a hybridization temperature (Tm) of an oligonucleotide sequence including the first nucleotide in the direction of 5' end from a nucleotide corresponding to the target site through 5' end nucleotide and an oligonucleotide sequence complementary thereto and a hybridization temperature (Tm) of an oligonucleotide sequence including the first nucleotide in the direction of 3' end from the nucleotide corresponding to the target site through 3' end nucleotide and an oligonucleotide sequence complementary thereto; and selecting an oligonucleotide probe having the difference of 0-5°C.

In the present invention, a method of calculating a hybridization temperature of an oligonucleotide having a known sequence is well known in the art. In connection with the method of calculating a hybridization temperature, John SantaLuchia, Jr. has repeatedly reported the experimental results of reactions of nucleotides since 1996 and the hybridization temperatures (Tm) according to each case has been broadly used. Also, a commercially available program visual OMP (available from DNA software, Inc., USA) or an opened public website MELTSIM (http://bioinformatics.org/meltsim/), etc. may be used to calculate the hybridization temperatures.

In the method of the present invention, factors that are commonly considered in the design of a probe may be considered in addition to the difference in the hybridization temperature. Such consideration factors include thermodynamic variables including a hybridization temperature with a target nucleic acid, a length, the formation of a homodimer in a molecule, the presence of a sequence homology with other polynucleotide probe, and a position of a target site.

The present invention will now be described in greater detail with reference to the following examples. The following examples are for illustrative purposes and are not intended to limit the scope of the invention.

### Examples

### Comparative Example: Design of an oligonucleotide probe according to a conventional method

In this Comparative Example, an oligonucleotide probe was designed according to a conventional method of designing an oligonucleotide probe.

In the design of the probe, a thermodynamic variable for the entire sequence of an oligonucleotide, including a hybridization temperature, a target site corresponding to the central of the probe, and a minimization of nonspecific binding through a search for hybridization were considered.

First, candidate probe sequences including a mutant site to be analyzed and having a length of 18-28 bp were selected. Then, hybridization simulation reactions between each of the probes and target samples were performed to select the probes having Tm of 65-80°C. The selected probes were subject to a hybridization reaction with other target sequence site except for a predetermined site and the probes having homology of 50% or greater were excluded. Also, these probes were subject to an investigation regarding a possibility of forming a homodimer in a probe molecule, and the probes having the possibility of forming a homodimer were excluded. Among these, the probes having a content of GC of 20-80% were selected. The probes in which a difference between Tm obtained from hybridization with a perfectly complementary sequence (perfect match) and Tm obtained from hybridization with a noncomplementary sequence of a target site (mismatch) was 3 or greater were selected. Among the selected probes, the probe having the greatest difference between the perfect match Tm and the mismatch Tm was selected.

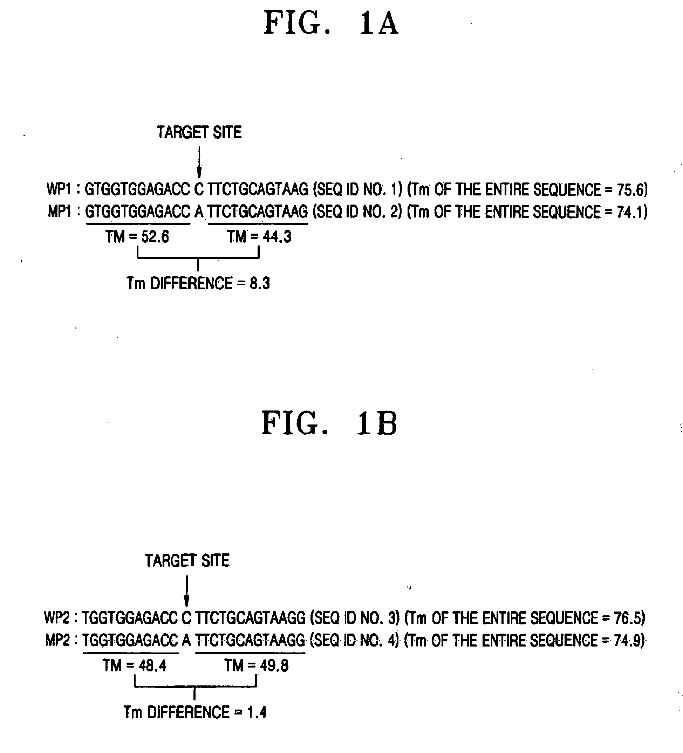

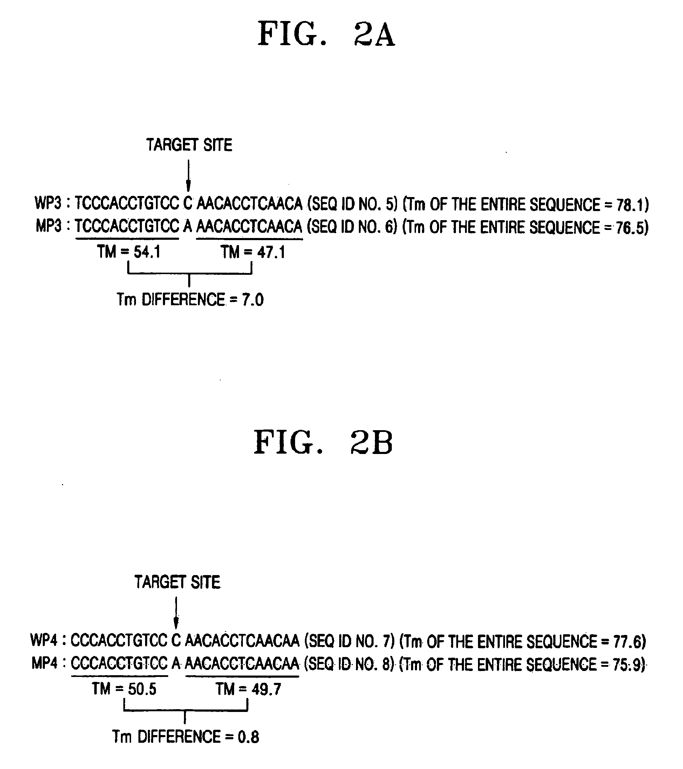

As a result, when a nucleotide site complementary to the 342^{nd} C of a target sample E01-11 (SEQ ID NO. 9) was used as a target site, a wild type probe (WP1: SEQ ID NO. 1) that was perfectly matched and a mutant type probe (MP1: SEQ ID NO. 2) that was not matched to only the target site were obtained. A difference between Tm of a sequence in the direction of 5' end and Tm of a sequence in the direction of 3' end, based on nucleotide sites of WP1 and MP1 corresponding to the target site was 8.3 (see FIG. 1A). Also, when a nucleotide site complementary to the 147th C of a target sample E02-22 (SEQ ID NO. 10) was used as a target site, a wild type probe (WP3: SEQ ID NO. 5) that was perfectly matched and a mutant type probe (MP3: SEQ ID NO. 6) that was not matched to only the target site were obtained. A difference between Tm of a sequence in the direction of 5' end and Tm of a sequence in the direction of 3' end, based on nucleotide sites of WP3 and MP3 corresponding to the target site was 7.0 (see FIG. 2A).

Although a difference between Tm of the entire sequence of WP1 and Tm of the whole sequence of MP1 is only 1.5, the difference between Tm of the sequence in the direction of 5' end and Tm of the sequence in the direction of 3' end, based on the nucleotide site corresponding to the target site is 8.3 which is relatively great. Thus, there is a possibility of nonspecific binding. Also, although a difference between Tm of the whole sequence of WP3 and Tm of the whole sequence of MP3 is only 1.6, the difference between Tm of the sequence in the direction of 5' end and Tm of the sequence in the direction of 3' end, based on the nucleotide site corresponding to the target site is 7.0 which is relatively great. Thus, there is a possibility of nonspecific binding. Since the nonspecific binding has a lower binding force compared to the specific binding, it causes a fluorescence intensity resulting from the hybridization to be lowered. Also, the nonspecific binding prevents specific binding with a target nucleic acid.

Thus, according to the conventional method of designing the oligonucleotide probe, binding specificity is lowered.

### Example 1: Design of an oligonucleotide probe according to the present invention

In this Example, besides the factors considered in Comparative Example, Tm of an oligonucleotide in the direction of 5' end and Tm of an oligonucleotide in the direction of 3' end, based on the nucleotide corresponding to the target site were calculated and the values were compared to select oligonucleotide probes having the smallest difference possible. Specifically, the same procedures as in the Comparative Example were performed except that hybridization reactions of all of the probes and target samples were performed to select the probes having Tm of 65-80°C. and then the probes in which a difference between Tm of a right side and Tm of a left side based on a target site, for example, a mutant site, was 5 or less, were further selected.

As a result, when a nucleotide site complementary to the 342^{nd} C of a target sample E01-11 (SEQ ID NO. 9) was used as a target site, a wild type probe (WP2: SEQ ID NO. 3) that was perfectly matched and a mutant type probe (MP2: SEQ ID NO. 4) that was not matched to only the target site were obtained. A difference between Tm of a sequence in the direction of 5' end and Tm of a sequence in the direction of 3' end, based on nucleotide sites of WP2 and MP2 corresponding to the target site was 1.4 (see FIG. 1B). Also, when a nucleotide site complementary to the 147th C of a target sample E02-22 (SEQ ID NO. 10) was used as a target site, a wild type probe (WP4: SEQ ID NO. 7) that was perfectly matched and a mutant type probe (MP4: SEQ ID NO. 8) that was not matched to only the target site were obtained. A difference between Tm of a sequence in the direction of 5' end and Tm of a sequence in the direction of 3' end, based on nucleotide sites of WP4 and MP4 corresponding to the target site was 0.8 (see FIG. 2B).

A difference between Tm of the whole sequence of WP2 and Tm of the whole sequence of MP2 is only 1.6 and the difference between Tm of the sequence in the direction of 5' end and Tm of the sequence in the direction of 3' end, based on the nucleotide site corresponding to the target site is 1.4 which is relatively small. Thus, a possibility of nonspecific binding is low. Also, a difference between Tm of the whole sequence of WP4 and Tm of the whole sequence of MP4 is only 1.7 and the difference between Tm of the sequence in the direction of 5' end and Tm of the sequence in the direction of 3' end, based on the nucleotide site corresponding to the target site is 0.8 which is relatively small. Thus, a possibility of nonspecific binding is low. Since a ratio of nonspecific binding becomes relatively low, a fluorescence intensity resulting from hybridization will increase when compared with the probe designed according to the conventional method.

### Example 2: Effect of the oligonucleotide probe of the present application on the result of hybridization

In this Example, oligonucleotide probes designed in the Comparative Example and Example 1, i.e., the probes complementary to the wild type target nucleic acids WP1, WP2, WP3, and WP4 and the probes complementary to the mutant type target nucleic acids MP1, MP2, MP3, and MP4 were immobilized on a microarray and hybridization reactions were performed, followed by an analyses of the results.

First, the above probes were immobilized on a substrate as probes for the target nucleic acids (E01-11 or E02-22) to complete the microarray. The probes WP1 and MP1, and the probes WP3 and MP3 were immobilized on a control microarray in the same manner. A microarray of the present invention was prepared using a spotting solution that was obtained by mixing the probes WP2 and MP2 and the probes WP4 and MP4 having amine groups with a hydrogel using a polyethylenglycol (PEG) having an epoxy group. The spotting solution was spotted on the surface of the glass, which was surface-treated so as to have amine groups, using a biorobot printer (model PixSys 5500, available from Cartesian Technologies, Inc., CA, USA), and then the glass was left in a humid incubator at 37°C for 4 hours. Then, a process required for controlling background noise was performed. That is, to prevent the target nucleic acid from attaching to the surface of the glass, a reaction was performed to allow amine groups on the unspotted surface of the glass to be negatively charged and the resultant was stored in a drier.

The target nucleic acid was labeled with a fluorescent material. In this Example, Cy3-dUTP was used as the fluorescent material and a body or both ends of the target nucleic acid was or were labeled.

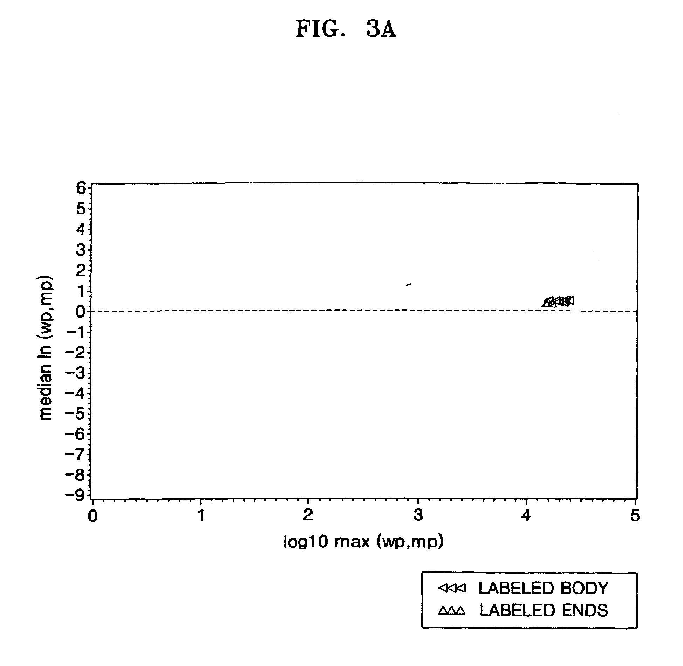

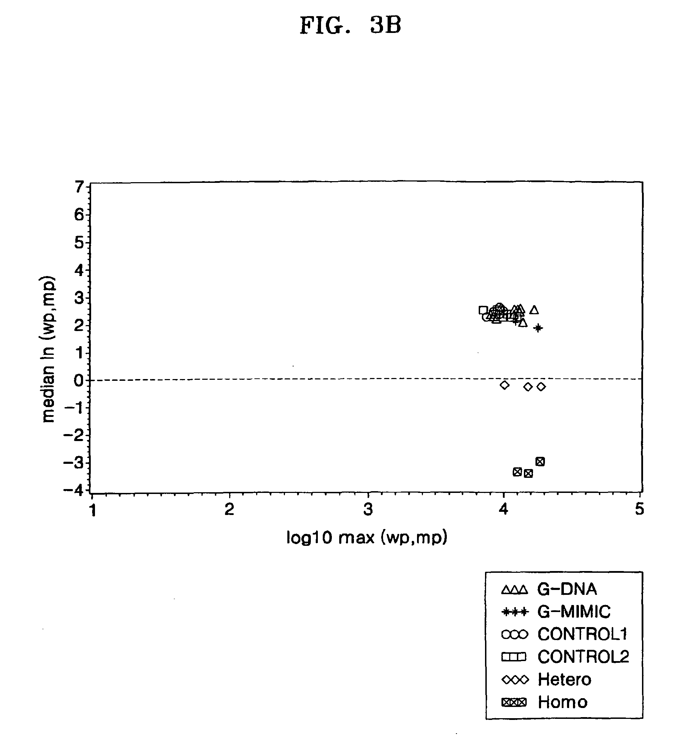

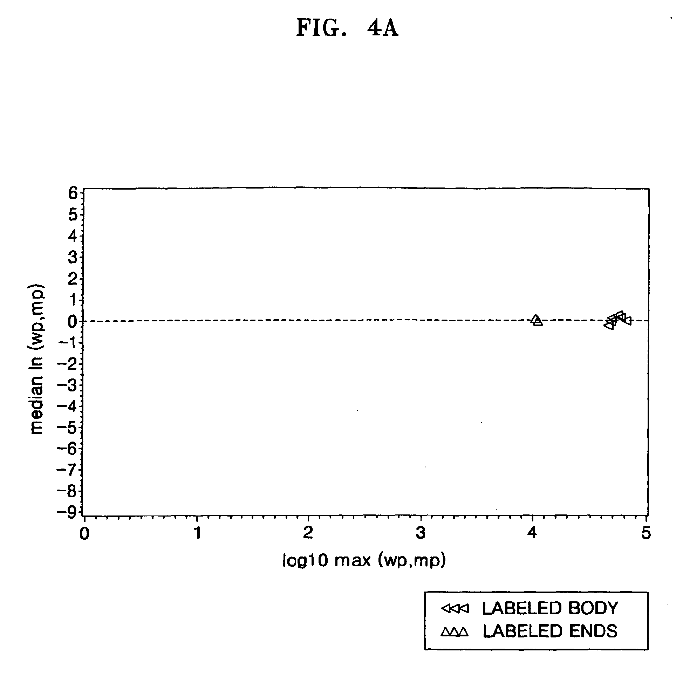

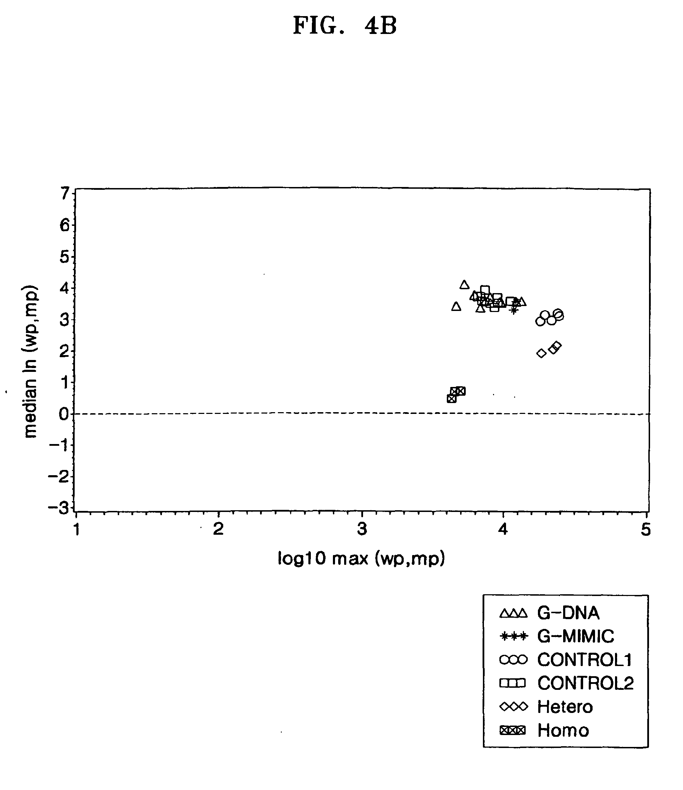

Hybridizations of the target nucleic acid and the probe were performed by reacting the target nucleic acid with a concentration of 20 nM in a 0.1 % 6 × SSPET (saline sodium phosphate EDTA buffer containing 0.1 % Trition X-100) solvent with the probe on the microarray at 37°C for 16 hours. Then, the microarray was washed with 0.05% 6 × SSPET and 0.05% 3 × SSPET at room temperature for 5 minutes each, dried at room temperature for 5 minutes, and then scanned. The scanning was performed using Axon scanner (model GenePix 4000B, available from Axon Instrument, Inc., CA, USA) and GenePix Pro 3.0 program (available from Axon Instrument, Inc., CA, USA) was used as a program for interpreting the scanning data so as to calculate a ratio component and an intensity component. The results are illustrated as MA graphs in FIGS. 3 and 4. Each data was obtained through hybridization of a normal type target nucleic acid with about 100 chips and hybridization of a mutant type target nucleic acid with about 30 chips. In FIGS. 3 and 4, the x axis represents the intensity component (A) and the y axis represents the ratio component (M). The ratio component (M) is obtained by applying a natural logarithm to a value of the ratio (r) = (a hybridization intensity of a mutant type probe (WP)/a hybridization intensity of a wild type probe (MP)). The mutant type probe is a probe that specifically hybridizes with a nucleic acid sequence including a mutant and includes MP1 (SEQ ID NO. 2), MP2 (SEQ ID NO. 4), MP3 (SEQ ID NO. 6), and MP4 (SEQ ID NO. 8). The wild type probe is a probe that specifically hybridizes with a mutant type nucleic acid sequence and includes WP1 (SEQ ID NO. 1), WP2 (SEQ ID NO. 3), WP3 (SEQ ID NO. 5), and WP4 (SEQ ID NO. 7). The intensity component (A) is obtained by applying natural logarithm to the maximum value of the hybridization intensity of the wild type probe (WP) and the hybridization intensity of the mutant type probe (MP).

FIG. 3A is a MA graph illustrating the result of hybridizing genome DNA derived from a normal human on a control microarray on which the probes WP1 and MP1 designed according to the conventional method are immobilized. FIG. 3B is a MA graph illustrating the results of hybridizing a target nucleic acid on a microarray on which the oligonucleotide probes WP2 and MP2 designed according to the method of the present invention are immobilized. Also, FIG. 4A is a MA graph illustrating the results of hybridizing genome DNA derived from a normal human on the control microarray on which the probes WP3 and MP3 designed according to the conventional method are immobilized. FIG. 4B a MA graph illustrating the results of hybridizing a target nucleic acid on a microarray on which the oligonucleotide probes WP4 and MP4 designed according to the method of the present invention are immobilized. In FIGS. 3B and 4B, G-DNA is the results of hybridization using genome DNA derived from a normal human as a target nucleic acid, G-MIMIC is the results of using synthesized G-DNA, and Hetero and Homo are the results of using genome DNAs derived from humans having a hetero type mutant and a homo-type mutant, respectively.

Referring FIGS. 3A, 3B, 4A and 4B, when using the oligonucleotide probe designed according to the method of the present invention, a ratio of fluorescence intensity of the mutant type probe to that of the wild type probe increases although fluorescence intensities are maintained at similar values. That is, a difference in the fluorescence intensity is increased by using the oligonucleotide probe designed according to the method of the present invention, and thus specific detection can be easily performed.

According to the oligonucleotide probe, a nonspecific reaction can be diminished in a hybridization reaction with a target nucleic acid. Thus, the oligonucleotide probe can be used by being immobilized on a substrate of a DNA microarray.

According to the microarray, since an oligonucleotide probe immobilized on a substrate has a small difference between a hybridization temperature of a sequence in the direction of 5' end from a nucleotide corresponding to a target site and a sequence complementary thereto and a hybridization temperature of a sequence in the direction of 3' end from the nucleotide corresponding to the target site and a sequence complementary thereto, strong hybridization signals can be obtained in the analysis method using the microarray.

In addition, according to the method of designing an oligonucleotide probe having improved binding specificity to a target site of the present invention, the oligonucleotide probe having improved binding specificity to a target site can be prepared.
## Claims

1. A method of designing an oligonucleotide probe for analyzing the type of gene of a target nucleic acid, said probe having enhanced binding specificity to a target site, which is a position of a certain nucleotide of which a variation is found in the target nucleic acid, the method comprising:
calculating a difference between a hybridization temperature (Tm) of an oligonucleotide sequence including the first nucleotide in the direction of 5' end from the nucleotide corresponding to the target site through 5' end nucleotide and an oligonucleotide sequence complementary thereto and a hybridization temperature (Tm) of an oligonucleotide sequence including the first nucleotide in the direction of 3' end from the nucleotide corresponding to the target site through 3' end nucleotide and an oligonucleotide sequence complementary thereto; and
selecting an oligonucleotide probe having the temperature difference of not greater than 5 DEG C.

2. A method of preparing an oligonucleotide probe for analyzing the type of gene of a target nucleic acid, said probe having enhanced binding specificity to a target site, which is a position of a certain nucleotide of which a variation is found in the target nucleic acid, the method comprising:
calculating a difference between a hybridization temperature (Tm) of an oligonucleotide sequence including the first nucleotide in the direction of 5' end from a nucleotide corresponding to the target site through 5' end nucleotide and an oligonucleotide sequence complementary thereto and a hybridization temperature (Tm) of an oligonucleotide sequence including the first nucleotide in the direction of 3' end from the nucleotide corresponding to the target site through 3' end nucleotide and an oligonucleotide sequence complementary thereto;
selecting an oligonucleotide probe having the temperature difference of not greater than 5 DEG C, and
obtaining the oligonucleotide probe.

## Patentansprüche

1. Verfahren zum Entwerfen einer Oligonukleotidsonde für die Analyse des Gentyps von einer Zielnukleinsäure, wobei die Sonde eine erhöhte Bindungspezifität mit einer Zielstelle, welche die Position eines bestimmten Nukleotids ist, von dem eine Variation in der Zielnukleinsäure gefunden wurde, aufweist, wobei das Verfahren umfasst:
Berechnen einer Differenz zwischen einer Hybridisierungstemperatur (Tm) von einer Oligonukleotidsequenz, die vom ersten Nukleotid, das in Richtung des 5'-Endes neben dem Nukleotid der Zielstelle liegt, bis zum Nukleotid am 5'-Ende reicht, und einer dazu komplementären Nukleotidsequenz, und einer Hybridisierungstemperatur (Tm) von einer Nukleotidsequenz, die vom ersten Nukleotid, das in Richtung des 3'-Endes neben dem Nukleotid der Zielstelle liegt, bis zum Nukleotid am 3'-Ende reicht, und einer dazu komplementären Oligonukleotidsequenz; und
Auswählen einer Oligonukleotidsonde mit einer Temperaturdifferenz von nicht größer als 5° C.

2. Verfahren zum Herstellen einer Oligonukleotidsonde für die Analyse des Gentyps von einer Zielnukleinsäure, wobei die Sonde eine erhöhte Bindungspezifität mit einer Zielstelle, welche die Position eines bestimmten Nukleotids ist, von dem eine Variation in der Zielnukleinsäure gefunden wurde, aufweist, wobei das Verfahren umfasst:
Berechnen einer Differenz zwischen einer Hybridisierungstemperatur (Tm) von einer Oligonukleotidsequenz, die vom ersten Nukleotid, das in Richtung des 5'-Endes neben dem Nukleotid der Zielstelle liegt, bis zum Nukleotid am 5'-Ende reicht, und einer dazu komplementären Nukleotidsequenz, und einer Hybridisierungstemperatur (Tm) von einer Nukleotidsequenz, die vom ersten Nukleotid, das in Richtung des 3'-Endes neben dem Nukleotid der Zielstelle liegt, bis zum Nukleotid am 3'-Ende reicht, und einer dazu komplementären Oligonukleotidsequenz;
Auswählen einer Oligonukleotidsonde mit einer Temperaturdifferenz von nicht größer als 5° C, und
Erhalten der Oligonukleotidsonde.

## Revendications

1. Méthode de conception d'une sonde oligonucléotidique pour l'analyse du type de gène d'un acide nucléique cible, ladite sonde ayant une spécificité de liaison accrue pour un site cible, qui est une position d'un certain nucléotide dont une variation se trouve dans l'acide nucléique cible, ladite méthode consistant à :
calculer une différence entre une température d'hybridation (Tm) d'une séquence oligonucléotidique comprenant le premier nucléotide en direction de l'extrémité 5' à compter du nucléotide correspondant au site cible jusqu'au nucléotide de l'extrémité 5' et d'une séquence oligonucléotidique complémentaire de celle-ci et une température d'hybridation (Tm) d'une séquence oligonucléotidique comprenant le premier nucléotide en direction de l'extrémité 3' à compter du nucléotide correspondant au site cible jusqu'au nucléotide de l'extrémité 3' et d'une séquence oligonucléotidique complémentaire de celle-ci ; et
sélectionner une sonde oligonucléotidique ayant la différence de températures qui ne dépasse pas 5 °C.

2. Méthode de préparation d'une sonde oligonucléotidique pour l'analyse du type de gène d'un acide nucléique cible, ladite sonde ayant une spécificité de liaison accrue pour un site cible, qui est une position d'un certain nucléotide dont une variation se trouve dans l'acide nucléique cible, ladite méthode consistant à :
calculer une différence entre une température d'hybridation (Tm) d'une séquence oligonucléotidique comprenant le premier nucléotide en direction de l'extrémité 5' à compter d'un nucléotide correspondant au site cible jusqu'au nucléotide de l'extrémité 5' et d'une séquence oligonucléotidique complémentaire de celle-ci et une température d'hybridation (Tm) d'une séquence oligonucléotidique comprenant le premier nucléotide en direction de l'extrémité 3' à compter du nucléotide correspondant au site cible jusqu'au nucléotide de l'extrémité 3' et d'une séquence oligonucléotidique complémentaire de celle-ci ;
sélectionner une sonde oligonucléotidique ayant la différence de températures qui ne dépasse pas 5 °C, et
obtenir la sonde oligonucléotidique.


< 110> Samsung Electronics Co. Ltd.
<120> A polynucleotide probe having enhanced binding specificity, a microarray having the probe and a method for designing the probe
<130> PN053860
<160> 10
<170> Kopatentln 1.71
<210> 1
   <211> 25
   <212> DNA
   <213> Artificial Sequence
<220>
   <223> WP1 probe
<400> 1
   gtggtggaga cccttctgca gtaag 25
<210> 2
   <211> 25
   <212> DNA
   <213> Artificial Sequence
<220>
   <223> MP1 probe
<400> 2
   gtggtggaga ccattctgca gtaag 25
<210> 3
   <211> 25
   <212> DNA
   <213> Artificial Sequence
<220>
   <223> WP2 probe
<400> 3
   tggtggagac ccttctgcag taagg 25
<210> 4
   <211> 25
   <212> DNA
   <213> Artificial Sequence
<220>
   <223> MP2 probe
<400> 4
   tggtggagac cattctgcag taagg 25
<210> 5
   <211> 25
   <212> DNA
   <213> Artificial Sequence
<220>
   <223> WP3 probe
<400> 5
   tcccacctgt cccaacacct caaca 25
<210> 6
   <211> 25
   <212> DNA
   <213> Artificial Sequence
<220>
   <223> MP3 probe
<400> 6
   tcccacctgt ccaaacacct caaca 25
<210> 7
   <211> 25
   <212> DNA
   <213> Artificial Sequence
<220>
   <223> WP4 probe
<400> 7
   cccacctgtc ccaacacctc aacaa 25
<210> 8
   <211> 25
   <212> DNA
   <213> Artificial Sequence
<220>
   <223> MP4 probe
<400> 8
   cccacctgtc caaacacctc aacaa 25
<210> 9
   <211> 419
   <212> DNA
   <213> Homo sapiens
<400> 9
   cgtggccctg tggcagccga gccatggttt ctaaactgag ccagctgcag acggagctcc 60
   tggcggccct gctcgagtca gggctgagca aagaggcact gatccaggca ctgggtgagc 120
   cggggcccta cctcctggct ggagaaggcc ccctggacaa gggggagtcc tgcggcggcg 180
   gtcgagggga gctggctgag ctgcccaatg ggctggggga gactcggggc tccgaggacg 240
   agacggacga cgatggggaa gacttcacgc cacccatcct caaagagctg gagaacctca 300
   gccctgagga ggcggcccac cagaaagccg tggtggagac ccttctgcag taaggagccc 360
   tgccccgtcc ccgctcccag gagagcctag aggggccccc ctcagctcct aacgagccc 419
<210> 10
   <211> 326
   <212> DNA
   <213> Homo sapiens
<400> 10
   cccttgctga gcagatcccg tccttgccct ctcccaggga ggacccgtgg cgtgtggcga 60
   agatggtcaa gtcctacctg cagcagcaca acatcccaca gcgggaggtg gtcgatacca 120
   ctggcctcaa ccagtcccac ctgtcccaac acctcaacaa gggcactccc atgaagacgc 180
   agaagcgggc cgccctgtac acctggtacg tccgcaagca gcgagaggtg gcgcagcgta 240
   agtaatgacc ctaccccgca tcttccctgg gagggcccag gactctcccc taactcatag 300
   gtgggggctg gaagcttcac catccc 326